(19) Europäisches Patentamt
European Patent Office
Office européen des brevets

(11) Veröffentlichungsnummer: **0 293 739 B1**

(12) **EUROPÄISCHE PATENTSCHRIFT**

(45) Veröffentlichungstag der Patentschrift: **14.11.90**

(51) Int. Cl.[5]: **C07C 31/135,** C07C 29/19, C07C 41/28, C07C 43/115

(21) Anmeldenummer: **88108356.2**

(22) Anmeldetag: **26.05.88**

(54) **Verfahren zur Herstellung von 4-Isopropyl-cyclohexylmethanol bzw. dessen Alkylethern.**

(30) Priorität: **03.06.87 DE 3718564**

(43) Veröffentlichungstag der Anmeldung: **07.12.88 Patentblatt 88/49**

(45) Bekanntmachung des Hinweises auf die Patenterteilung: **14.11.90 Patentblatt 90/46**

(84) Benannte Vertragsstaaten: **CH DE FR GB LI**

(56) Entgegenhaltungen:
**EP-A- 0 054 699**
**EP-A- 0 059 373**
**EP-A- 0 222 984**
**DE-A- 3 224 033**

**M. HUDLICKY: "Reductions in organic chemistry", Ellis Horwood Ltd., 1984, Chichester, GB**
**P.N. RYLANDER: "Hydrogenation methods",Kapitel 13, Academic Press, 1985, London, GB**

(73) Patentinhaber: **BASF Aktiengesellschaft, Carl-Bosch-Strasse 38, D-6700 Ludwigshafen(DE)**

(72) Erfinder: **Degner, Dieter, Dr., Kurpfalzstrasse 8, D-6701 Dannstadt-Schauernheim(DE)**
Erfinder: **Gramlich, Walter, Dr., Auf der Hoehe 11, D-6803 Edingen-Neckarhausen(DE)**
Erfinder: **Lengsfeld, Wolfgang, Dr., Woogstrasse 46, D-6703 Limburgerhof(DE)**
Erfinder: **Schuster, Ludwig, Dr., Weinheimer Strasse 44, D-6703 Limburgerhof(DE)**

EP 0 293 739 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

In der DE-0S 24 27 609 (bzw. US-PS 3 993 604) werden neue alicyclische Verbindungen der allgemeinen Formel III

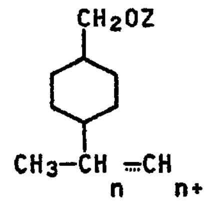

III

beschrieben, welche in der durch die gestrichelten Linien gekennzeichneten Stellung eine einfache Bindung oder eine Doppelbindung aufweisen, und in welcher n die Zahl 0 oder 1 und Z Wasserstoff, eine Alkyl- oder eine Acylgruppe mit 1 bis 6 Kohlenstoffatomen darstellt.

Die wichtigste Verbindung in dieser Klasse ist das unter dem Handelsnamen Mayol® vertriebene 4-Isopropyl-cyclohexylmethanol (Z = H, n = 1, gestrichelte Linie = Einfachbindung) sowie dessen Ethylether ($Z = C_2H_5$, n = 1, gestrichelte Linie = Einfachbindung).

In den aufgeführten Patentschriften wird ein Verfahren zur Herstellung des 4-Isopropyl-cyclohexylmethanols beschrieben, das auf einer Kernhydrierung von Cuminaldehyd der Formel IV

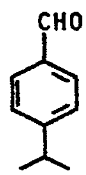

IV

beruht. Nachteilig an diesem Verfahren ist, daß der Cuminaldehyd nur auf aufwendige Weise zugängig ist.

Auch die Herstellung der Ether der Formel III (Z = Alkyl) gemäß loc.cit. ist sehr unbefriedigend. Sie erfolgt aus den entsprechenden Alkoholen durch Umsetzung mit Natriumhydrid in Dimethylsulfoxid (DMSO) und anschließender Alkylierung mit Alkylhalogeniden. Diese Methode läßt sich im Labormaßstab sicherlich gut realisieren, ist aber großtechnisch aufgrund des in der Handhabung problematischen Natriumhydrid/DMSO-Gemisches, aufgrund der durch den Umsatz mit Alkylhalogeniden entstehenden molaren Salzmengen (NaBr) sowie des bei der Waschung ins Abwasser gelangenden DMSO heutzutage nur unter hohen Auflagen und Kosten durch führbar. Weiterhin ist das Arbeiten mit Alkylhalogeniden aus toxikologischen Gründen nicht ganz unproblematisch, da diese z.T. ein cancerogenes Potential aufweisen.

Es war daher die Aufgabe der Erfindung ein Verfahren für die Herstellung des 4-Isopropyl-cyclohexylmethanols bzw. dessen Alkylethern zu finden, bei dem man von leichter zugänglichen und daher billigeren Ausgangsverbindungen ausgehen kann und das sich möglichst einfach und ohne großes toxikologisches Risiko realisieren läßt.

Es wurde nun überraschenderweise gefunden, daß man den neuen und neuerdings gemäß der nicht vorveröffentlichten Patentschrift DE-OS .. .. (P 36 44 076.0) recht leicht auf elektrochemischem Wege aus p-Cymol erhältlichen 4-(1-Alkoxy-1-methyl-ethyl)-benzaldehyd durch katalytische Hydrierung oberhalb 100°C in nahezu quantitativer Ausbeute unter Abspaltung von Alkanolen (aus der Alkoxigruppe und Wasserstoff), Hydrierung der dabei gebildeten Doppelbindung, Hydrierung des aromatischen Kernes sowie der Aldehydgruppe in das begehrte 4-Isopropyl-cyclohexylmethanol überführen kann. Setzt man anstelle des genannten Benzaldehyds dessen Dialkylacetal als Ausgangsverbindung ein, so erhält man den entsprechenden Alkylether in ebenfalls fast quantitativer Ausbeute unter Abspaltung von zwei Molen Alkanol (d.h. Alkoxigruppen und Wasserstoff), Hydrierung der gebildeten Doppelbindung und Kernhydrierung.

Gegenstand der Erfindung ist daher ein Verfahren zur Herstellung von 4-Isopropyl-cyclohexylmethanol bzw. dessen Alkylethern der allgemeinen Formel I

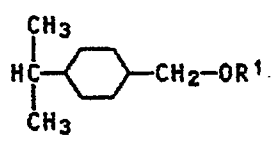

(I)

der $R^1$ für H oder einen $C_1$- bis $C_4$-Alkylrest steht, dadurch gekennzeichnet, daß man den neuen 4-(1-Alkoxy-1-methyl-ethyl)-benzaldehyd bzw. sein Dialkylacetal der allgemeinen Formel II

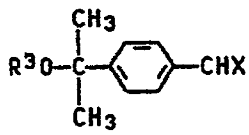 (II)

in der X für ein Sauerstoffatom (IIa) oder für zwei $R^2O$-Gruppen (IIb) steht und $R^2$ und $R^3$ einen $C_1$- bis $C_4$-Alkylrest bedeuten, in Gegenwart von Edelmetallen der Gruppe VIII des Periodensystems unter Wasserstoffdrücken von 50 bis 350 bar, vorzugsweise 150 bis 300 bar, auf Temperaturen von 100 bis 250°C, vorzugsweise 130 bis 200°C erhitzt.

Die als Ausgangsstoffe verwendeten neuen 4-(1-Alkoxy-1-methyl-ethyl)-benzaldehyddiacetale IIb erhält man auf relativ einfache Weise durch elektrochemische Oxidation der entsprechenden 4-(1-Alkoxy-1-methyl-ethyl)-toluole in Gegenwart eines niederen Alkanols. Durch Hydrolyse mit Wasser erhält man aus diesen Diacetalen IIb die Benzaldehyde IIa in sehr guten Ausbeuten.

Als Edelmetalle der Gruppe VIII des Periodensystems sind im wesentlichen die bekannten Hydrierkatalysatoren wie Nickel (in Form von Raney-Nickel), Palladium, Platin, Rhodium und Ruthenium geeignet. Als besonders vorteilhaft hat sich Ruthenium erwiesen. Im allgemeinen verwendet man die Edelmetalle aufgetragen auf Katalysatorträgermaterial, wie Aluminiumoxid oder Aktivkohle in Mengen von 0,5 bis 10 Gew.%, bezogen auf das Trägermaterial. Es lassen sich jedoch auch die reinen Metalle oder Metallverbindungen einsetzen.

Die Reaktionsbedingungen können hinsichtlich Katalysatoren, Lösungsmitteln, Reaktionstemperatur, Wasserstoffdruck und Reaktionszeit in relativ weiten Grenzen variiert werden. Bevorzugt werden Temperaturen zwischen 100 und 250°C angewandt. Unter 100°C findet praktisch keine Alkanolabspaltung statt. Die Wasserstoffdrücke liegen normalerweise zwischen 50 und 350 bar, bevorzugt arbeitet man zwischen 150 und 300 bar.

Als Lösungsmittel für die Hydrierung können beispielsweise Alkanole, wie Methanol oder Ethanol; Ether, wie Tetrahydrofuran; Kohlenwasserstoffe, wie Pentan; und Säuren wie Essigsäure eingesetzt werden; die Hydrierung kann jedoch auch ohne Lösungsmittel durchgeführt werden.

Bei der Hydrierung von 4-(1-Alkoxy-1-methyl-ethyl)-benzaldehyd (IIa) entstehen Gemische der cis- und trans-Isomeren; bei Anwendung des bevorzugten Ruthenium-Katalysators liegt das cis/trans-Verhältnis bei 60-70/40-30.

Man kann die Parameter der Reaktion so wählen, daß man die entstehenden Zwischenstufen

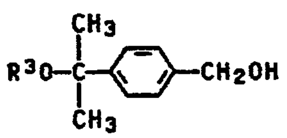 V 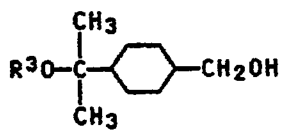 VI

4-(1-Alkoxy-1-methyl-ethyl)-benzylalkohol (V) sowie 4-(1-Alkoxy-1-methyl-ethyl)-cyclohexylmethanol (VI) isolieren kann, die jedoch olfaktorisch nicht von Bedeutung sind.

Auch die bei der Hydrierung aus den Acetalen IIb erhaltenen Ether der Formel I fallen als cis/trans-Isomere an. Das Isomerenverhältnis liegt auch hier bei cis/trans = 60-70/40-30.

Mit Hilfe des erfindungsgemäßen Verfahrens können die als Riechstoffe sehr begehrten Verbindungen der Formel I auf sehr elegante Weise aus neuen aber gut zugänglichen Ausgangsverbindungen hergestellt werden. Insbesondere wurden die für die Herstellung der Ether der Formel I gemäß dem zitierten Stand der Technik beschriebenen erheblichen Nachteile vermieden. Da bei dem erfindungsgemäßen Verfahren darüberhinaus die Dialkylacetale IIb die Vorstufen für die Herstellung der entsprechenden Aldehyde sind, werden zusätzlich zwei Verfahrensschritte eingespart. Die nachstehenden Beispiele sollen das Verfahren erläutern.

Beispiel 1

A. Elektrosynthese von 4-(1-Methoxy-1-methyl-ethyl)-benzaldehyddimethylacetal aus 4-(1-Methoxy-1-methyl-ethyl)-toluol

Apparatur: ungeteilte Zelle mit 5 Elektroden, Elektrodenabstand: 1,5 mm. Anoden: Graphit; Kathoden: Graphit.

In einem ersten Ansatz wurde für die Elektrolyse ein Elektrolyt mit folgender Zusammensetzung eingesetzt: 14,03 kg 4-(1-Methoxy-1-methyl-ethyl)-toluol, 0,63 kg $KSO_3C_6H_5$, 0,16 kg $C_6H_5SO_3H$, 62,2 kg

$CH_3OH$.

Die Elektrolyse wurde mit 11 F/Mol 4-(1-Methoxy-1-methyl-ethyl)-toluol bei einer Stromdichte von 3,6 $A/dm^2$ und der Temperatur von 26°C vorgenommen. Dabei wurde der Elektrolyt mit 800 l/h über einen Wärmetauscher durch die Zelle gepumpt.

In einem zweiten Ansatz wurde die Elektrolyse mit folgendem Elektrolyten analog durchgeführt.

Elektrolyt: 11,83 kg 4-(1-Methoxy-1-methyl-ethyl)-toluol, 0,68 kg $KSO_3C_6H_5$, 0,17 kg $C_6H_5SO_3H$, 67,7 kg $CH_3OH$.

Die beiden Elektrolyseausträge wurden nach Beendigung der Elektrolysen vereinigt und zusammen aufgearbeitet.

Aufarbeitung:

Zunächst wurde Methanol bei Normaldruck bis zu Sumpftemperaturen von 125°C abdestilliert. Der Rückstand wurde auf ca. 30°C abgekühlt und über eine Drucknutsche filtriert. Hierbei erhielt man 0,9 kg eines Salzes, das zusammen mit dem Methanol zur Elektrolyse zurückgeführt werden kann. Das Filtrat (43,3 kg) wurde dann bei 2 mbar Kopfdruck und 100 bis 120°C (Kopftemperaturen) rektifiziert. Hierbei erhielt man 0,17 kg 4-(1-Methoxy-1-methyl-ethyl)-toluol, 1,54 kg 1-Methoxy-methyl-4-(1-methoxy-1-methylethyl)-benzol und 20,88 kg 4-(1-Methoxy-1-methyl-ethyl)-benzaldehyddimethylacetal. Hieraus errechnet sich ein Umsatz, bezogen auf 4-(1-Methoxy-1-methyl-ethyl)-toluol, von 99,3 %, eine Ausbeute an 1-Methoxymethyl-4-(1-methoxy-1-methyl-ethyl)-benzol von 5 % und eine Ausbeute an 4-(1-Methoxy-1-methyl-ethyl)-benzaldehyddimethylacetal von 59,1 %. Die Selektivität für das Dimethylacetal beträgt 62,7 %. Die nicht umgesetzte Ausgangsverbindung und das 1-Methoxy-methyl-4-(1-methoxy-1-methyl-ethyl)-benzol können zur Elektrolyse rückgeführt werden.

B. Herstellung von 4-Isopropyl-cyclohexylmethyl-methylether

In einem Autoklaven wurde eine Lösung von 575 g (2,57 Mol) des gemäß A erhaltenen Ausgangsstoffes 4-(1-Methoxy-1-methyl-ethyl)-benzaldehyddimethylacetal in 600 ml Tetrahydrofuran (THF) in Gegenwart von 9 g Rutheniumhydroxid mehrfach mit Stickstoff und Wasserstoff gespült und dann bei einer Temperatur von 130°C und einem Wasserstoffdruck von 200 bar bis zur Druckkonstanz hydriert.

Nach Entfernen des Katalysators wurde zunächst das THF abdestilliert und danach der Rückstand bei 10 mbar fraktioniert destilliert.

Man erhielt 410 g (94 % Ausbeute) des als Stoff selbst noch nicht beschriebenen 4-Isopropyl-cyclohexylmethyl-methylethers als 65/35-cis/trans-Gemisch.

Kp. 42/0,3 mbar, $n_D^{25} = 1{,}4465$

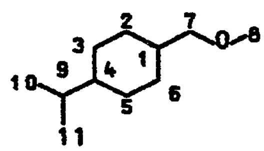

IR: 2955, 2922, 2869, 2855, 1449, 1385, 1197, 1117 $cm^{-1}$.

$^{13}C$-NMR (cis-Isomeres): δ = 75.67 ($C_7$,t), 58.72 ($C_8$,q), 43.05 ($C_4$,d), 34.70 ($C_1$,d), 30.52 ($C_9$,d), 26.59 ($C_6$ und $C_2$,t), 25.80 ($C_3$ und $C_5$,t), 20.28 ($C_{10}$ und $C_{11}$,q) ppm.

$^{13}C$-NMR (trans-Isomeres): δ = 79.00 ($C_7$,t), 58.72 ($C_8$,q), 44.36 ($C_4$,d), 38.34 ($C_1$,d), 33.0 ($C_9$,d), 30.27 ($C_6$ und $C_2$,t), 29.33 ($C_3$ und $C_5$,t), 19.86 ($C_{10}$ und $C_{11}$,q) ppm.

MS (m/e): $M^+$: 170 (0.1 %), 138 (38), 123 (12), 109 (26), 95 (100), 81 (28), 69 (54), 55 (32), 45 (50), 41 (35).

Beispiel 2

A. Herstellung von 4-(1-Methoxy-1-methyl-ethyl)-benzaldehyd aus 4-(1-Methoxy-1-methyl-ethyl)-benzaldehyddimethylacetal

In einem Rührkolben wurden 1,8 kg 4-(1-Methoxy-1-methyl-ethyl)-benzaldehyddimethylacetal und 1,8 kg Wasser unter Rühren ca. 1 Stunde unter Rückfluß zum Sieden (Sumpftemperatur 87°C) erhitzt. Danach kühlte man auf ca. 25°C ab, trennte die Phasen und destillierte die organische Phase bei 4 mbar Kopfdruck und 105 bis 108°C (Kopftemperaturen). Hierbei erhielt man 1314,6 g 4-(1-Methoxy-1-methylethyl)-benzaldehyd.

$^1H$-NMR: $(CH_3)_2C$-: 1,55 ppm (s), $-OCH_3$: 3,1 ppm (s), ArH: 7,6 ppm (d), 7,90 ppm (d), -CHO: 10,0 ppm (s), $n_D^{25} = 1{,}5264$

Dies entspricht einer Ausbeute von 97,3 %.

B. Herstellung von 4-Isopropyl-cyclohexylmethyl-methylether

In einem Autoklaven wurden 405 g (2,5 Mol) des gemäß A erhaltenen 4-(1-Methoxy-1-methyl-ethyl)-benzaldehyds in 200 ml THF in Gegenwart von 1 g Rutheniumhydroxid mehrfach mit Stickstoff und Wasserstoff gespült und dann bei einer Temperatur von 150°C und einem Wasserstoffdruck von 300 bar bis zur Druckkonstanz hydriert.

Nach Entfernen des Katalysators wurde zunächst das THF abdestilliert und danach der Rückstand bei 10 mbar fraktioniert destilliert. Man erhielt ein Diastereomerengemisch im Verhältnis cis/trans von 65/35.

Die beiden reinen cis- und trans-Isomeren ließen sich wie in DE-OS 24 27 609 beschrieben durch eine aufwendige Destillation (Drehbandkolonne, Labor-Sulzerkolonne) trennen und waren mit den in der Literatur [J.Org.Chem. 31, 3507 (1966)] angegebenen Daten identisch.

**Patentansprüche**

1. Verfahren zur Herstellung von 4-Isopropyl-cyclohexylmethanol bzw. dessen Alkylethern der allgemeinen Formel I

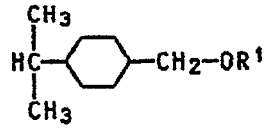

(I)

in der $R^1$ für H oder einen $C_1$- bis $C_4$-Alkylrest steht, dadurch gekennzeichnet, daß man 4-(1-Alkoxy-1-methyl-ethyl)-benzaldehyd bzw. sein Dialkylacetal der allgemeinen Formel II

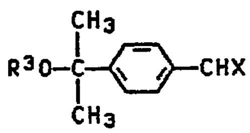

(II)

in der X für ein Sauerstoffatom (IIa) oder für zwei $R^2O$-Gruppen (IIb) steht und $R^2$ und $R^3$ einen $C_1$- bis $C_4$-Alkylrest bedeuten, in Gegenwart von Edelmetallen der Gruppe VIII des Periodensystems unter Wasserstoffdrücken von 50 bis 350 bar auf Temperaturen von 100 bis 250°C erhitzt.

2. Verfahren gemäß Anspruch 1, dadurch gekennzeichnet, daß man die Verbindung der Formel II auf Temperaturen von 130 bis 200°C erhitzt.

3. Verfahren gemäß Anspruch 1, dadurch gekennzeichnet, daß man die Verbindung der Formel II unter Wasserstoffdrucken von 150 bis 300 bar erhitzt.

4. Verfahren gemäß Anspruch 1, dadurch gekennzeichnet, daß man zur Herstellung von 4-Isopropylcyclohexylmethanol den 4-(1-Alkoxy-1-methyl-ethyl)-benzaldehyd in Gegenwart der Edelmetalle und Wasserstoff erhitzt.

5. Verfahren gemäß Anspruch 1, dadurch gekennzeichnet, daß man zur Herstellung eines 4-Isopropyl-cyclohexyl-methanolalkylethers ein 4-(1-Alkoxy-1-methyl-ethyl)-benzaldehyddialkylacetal in Gegenwart der Edelmetalle und Wasserstoff erhitzt.

6. Verfahren gemäß Anspruch 1, dadurch gekennzeichnet, daß man als Edelmetalle der Gruppe VIII des Periodensystems Nickel, Rhodium, Palladium, Platin oder Ruthenium verwendet.

7. Verfahren gemäß Anspruch 1, dadurch gekennzeichnet, daß man als Edelmetall der Gruppe VIII des Periodensystems Ruthenium verwendet.

**Claims**

1. A process for the preparation of 4-isopropylcyclohexylmethanol or its alkyl ethers of the formula I

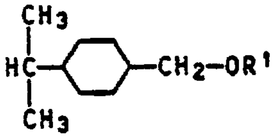

(I)

where $R^1$ is H or $C_1$–$C_4$-alkyl, wherein a 4-(1-alkoxy-1-methylethyl)-benzaldehyde or its dialkyl acetal of the formula II

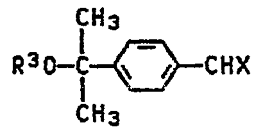

(II)

where X is oxigen (IIa) or is two $R^2O$ groups (IIb) and $R^2$ and $R^3$ are each $C_1$–$C_4$-alkyl, is heated to 100–250°C under a hydrogen pressure of from 50 to 350 bar in the presence of a noble metal of group VIII of the Periodic Table.

2. A process as claimed in claim 1, wherein a compound of the formula II is heated to 130–200°C.

3. A process as claimed in claim 1, wherein a compound of the formula II is heated under a hydrogen pressure of 150 to 300 bar.

4. A process as claimed in claim 1, wherein a 4-(1-alkoxy-1-methylethyl)-benzaldehyde is hêated in the presence of a noble metal and hydrogen for the preparation of 4-isopropylcyclohexylmethanol.

5. A process as claimed in claim 1, wherein a 4-(1-alkoxy-1-methylethyl)-benzaldehyde dialkyl acetal is heated in the presence of a noble metal and hydrogen for the preparation of a 4-isopropylcyclohexylmethyl alkyl ether.

6. A process as claimed in claim 1, wherein nickel, rhodium, palladium, platinum or ruthenium is used as the noble metal of group VIII of the Periodic Table.

7. A process as claimed in claim 1, wherein ruthenium is used as the noble metal of group VIII of the Periodic Table.

**Revendications**

1. Procédé de préparation de 4-isopropylcyclohexylméthanol ou ses esters alkyliques de la formule générale

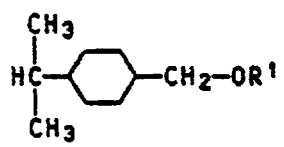

(I)

dans laquelle $R^1$ est mis pour H ou un reste alkyle en $C_1$ à $C_4$, caractérisé par le fait qu'on chauffe à des températures de 100 à 250 degrés C, sous une pression d'hydrogène de 50 à 350 bar, en présence de métaux nobles du groupe VIII du système périodique, du 4-(1-alcoxy-1-méthyl-éthyl)-benzaldehyde ou son dialkylacétal de la formule générale II

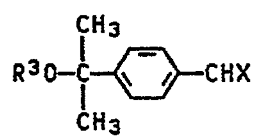

(II)

dans laquelle X est mis pour un atome d'oxygène (IIa) ou deux groupes $R^2O$ (IIb) et $R^2$ et $R^3$ représentent un reste alkyle en $C_1$ à $C_4$.

2. Procédé selon la revendication 1, caractérisé par le fait qu'on chauffe le composé de formule générale II à des températures de 130 à 200 degrés C.

3. Procédé selon la revendication 1, caractérisé par le fait qu'on chauffe le composé de formule il sous une pression d'hydrogène de 150 à 300 bar.

4. Procédé selon la revendication 1, caractérisé par le fait que, pour préparer du 4-isopropyl-cyclohexylméthanol, on chauffe le 4-(1-alcoxy-1-méthyl-éthyl)-benzaldehyde en présence de métaux nobles et d'hydrogène.

5. Procédé selon la revendication 1, caractérisé par le fait que, pour préparer un éther alkylique de 4-isopropylcyclohexylméthanol, on chauffe un dialkylacétal de 4-(1-alcoxy-1-méthyl-éthyl)-benzaldehyde en présence de métaux nobles est d'hydrogène.

6. Procédé selon la revendication 1, caractérisé par le fait qu'on utilise, comme métaux nobles du groupe VIII du système périodique, du nickel, rhodium, palladium, platine ou ruthénium.

7. Procédé selon la revendication 1, caractérisé par le fait qu'on utilise, comme métaux nobles du groupe VIII du système périodique, du ruthénium.